# EUROPÄISCHE PATENTSCHRIFT

(11) **EP 1 557 160 B1**
(45) Veröffentlichungstag und Bekanntmachung des Hinweises auf die Patenterteilung: **04.04.2007**
(21) Anmeldenummer: 04106537.6
(22) Anmeldetag: 14.12.2004
(51) Int. Cl.: A61K 8/04, A61Q 19/00, A61K 8/37, A61K 8/34, A61K 8/81, A61K 8/46, A61K 8/92

(54) **Schäumbare kosmetische O/W-Emulsion**
Foamable cosmetic o/w-emulsion
émulsions huile-dans-eau moussantes

(30) Priorität: 24.12.2003 DE 10361202
(43) Veröffentlichungstag der Anmeldung: 27.07.2005
(73) Patentinhaber: Beiersdorf AG, 20253 Hamburg (DE)
(72) Erfinder: Maurer, Peter, 24536, Neumünster (DE); Raschke, Thomas, 25421, Pinneberg (DE); Treu, Jens, 22844, Norderstedt (DE); Voigt, Nadine, 22455, Hamburg (DE)

(56) Entgegenhaltungen:
- DE-A1- 10 063 341
- US-B1- 6 579 907

## Beschreibung

Die vorliegende Erfindung betrifft eine kosmetische Emulsion.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement. Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 11 wasserlösliche, hydrophile.

Eine besonders weit verbreitete Form kosmetischer Emulsionen stellen (dünnflüssige) Öl-in-Wasser-Emulsionen (O/W-Emulsionen) dar.

Die Mehrzahl der O/W-Emulsionen haben jedoch den Nachteil, dass sie in der Regel gar nicht oder nur unzureichend aufschäumbar sind. Andere Emulsionen des Standes der Technik führen allenfalls zu ungleichmäßigen und grobblasigen Schäumen, die schnell wieder in sich zusammen fallen.

Es war deshalb die Aufgabe der vorliegenden Erfindung, die Mängel des Standes der Technik zu beseitigen und eine aufschäumbare O/W-Emulsion auf der Basis des Emulgators Glycerinmonostearat / PEG-40 Rizinusöl / Fettalkohol / Cetearylsulfat zu entwickeln, deren Schaum nach Produktentnahme eine feinporige Konsistenz aufweist wobei der Schaum über einen Zeitraum von mindestens 1 Minute stabil ist und 50% seines ursprünglichen Volumens bei feinporiger Schaumstruktur beibehält. Insbesondere sollte der Emulsion bei erhöhter Temperatur (40 °C) über einen Zeitraum von mindestens 3 Monaten lagerstabil sein, das heißt nach Produktentnahme einen wie oben beschrieben hochwertigen Schaum entwickeln, ohne das die Emulsion während der Lagerung bei 40 °C durch Ausfällungen oder Phasentrennungen zerstört wurde.

Überraschend gelöst wird die Aufgabe durch eine kosmetische O/W-Emulsion enthaltend eine Emulgatorkombination aus
a) Glycerylmonostearat in einer Konzentration von bis 0,1 bis 3,0 Gewichts-%,
b) Fettalkohol in einer Konzentration von 0,1 bis 4,0 Gewichts-%,
c) PEG-40 Rizinusöl in einer Konzentration von 0,1 bis 3,0 Gewichts-%,
d) Natriumcetylstearylsulfat in einer Konzentration von 0,01 bis 1,0 Gewichts-%
jeweils bezogen auf das Gesamtgewicht der Emulsion,
wobei die Ölphase der Emulsion dadurch gekennzeichnet ist, dass sie 1 bis 90 Gewichts-%, bezogen auf das Gesamtgewicht der Ölphase, unpolare Ölkomponenten enthält.

Die erfindungsgemäßen Emulsionen weisen auch im geschäumten Zustand eine ungewöhnlich hohe Lagerstabilität bei erhöhter Temperatur auf. Bei der Anwendung auf der Haut hinterlässt die Emulsion einen reichhaltigen, gehaltvollen sensorischen Eindruck.

Das erfindungsgemäße Glycerinmonostearat (INCI Glyceryl Stearate), ist unter der CAS-Nummmer CAS 123-94-4/11099-07-3/31566-31-1 in den Chemical Abstracts abgelegt. Erfindungsgemäß besonders bevorzugt wird es in einer Konzentration von 0,5 bis 1,5 Gewichts-%, bezogen auf das Gesamtgewicht der Zubereitung, eingesetzt.

Der erfindungsgemäß bevorzugte Fettalkohol ist Cetearylalkohol (Cetostearylalkohol, CAS 8005-44-5). Erfindungsgemäß besonders bevorzugt wird der Fettalkohol in einer Konzentration von 1 bis 3 Gewichts-%, bezogen auf das Gesamtgewicht der Zubereitung, eingesetzt.

Als PEG-40 Rizinusöl (INCI: PEG-40 castor oil) wird erfindungsgemäß das Polyethylenglykol(2000)ricinusöl (CAS 61791-12-6) eingesetzt. Erfindungsgemäß besonders bevorzugt wird es in einer Konzentration von 0,3 bis 1,0 Gewichts-%, bezogen auf das Gesamtgewicht der Zubereitung, eingesetzt.

Das erfindungsgemäße Natriumcetylstearylsulfat (INCI: sodium cetearyl sulfate, CAS 59186-41-3) ist ein Gemisch aus Cetyl- u. Stearylsuflat. Erfindungsgemäß besonders bevorzugt wird es in einer Konzentration von 0,1 bis 0,5 Gewichts-%, bezogen auf das Gesamtgewicht der Zubereitung, eingesetzt.

Die erfindungsgemäßen Substanzen Cetearylalkohol, PEG-40 Rizinusöl und Natriumcetylstearylsulfat können auch als Mischung eingesetzt werden. Entsprechende Rohstoffe sind unter dem Handelsnamen Emulgade F (Hersteller Firma COGNIS) verfügbar.

Erfindungsgemäß vorteilhafte Ausführungsformen der vorliegenden Erfindung sind dadurch gekennzeichnet, dass die Gesamtmenge an Emulgatoren a), b), c) und d) von 0,5 bis 8,0 Gewichts-% und besonders bevorzugt von 1,0 bis 5,0 Gewichts-%, jeweils bezogen auf das Gesamtgewicht der Emulsion, beträgt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Ölkomponenten angesehen, deren Grenzflächenspannung gegen Wasser weniger als 20 mN/m beträgt als unpolar solche, deren Grenzflächenspannung gegen Wasser mehr als 30 mN/m beträgt. Ölkomponenten mit einer Grenzflächenspannung gegen Wasser zwischen 20 und 30 mN/m werden im allgemeinen als mittelpolar bezeichnet.

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Bevorzugte Ölkomponenten im Sinne der vorliegenden Erfindung sind die im folgenden aufgelisteten Substanzen:

| **Hersteller** | **Handelsname** | **INCI-Name** | **Polarität [mN/m ]** |
|---|---|---|---|
| Total SA | Ecolane 130 | Cycloparaffin | 49,1 |
| Neste PAO N.V. (Lief. Hansen & Rosenthal) | Nexbase 2006 FG | Polydecene | 46.7 |
| Chemische Fabrik Lehrte | Polysynlane | Hydrogenated Polyisobutene | 44.7 |
| Wacker | Wacker Silikonöl AK 50 | Polydimethylsiloxan | 46,5 |
| EC Erdölchemie (Lieferant Bayer AG) | Solvent ICH | Isohexadecane | 43.8 |
| DEA Mineralöl (Lief. Hansen & Rosenthal) Tudapetrol | Pionier 2076 | Mineral Oil | 43.7 |
| DEA Mineralöl (Lief. Hansen & Rosenthal) Tudapetrol | Pionier 6301 | Mineral Oil | 43.7 |
| Wacker | Wacker Silikonöl AK 35 | Polydimethylsiloxan | 42,4 |
| EC Erdölchemie GmbH | Isoeikosan | Isoeikosan | 41.9 |
| Wacker | Wacker Silikonöl AK 20 | Polydimethylsiloxan | 40,9 |
| Condea Chemie | Isofol 1212 Carbonat | | 40,3 |
| Gattefossé | Softcutol O | Ethoxydiglycol Oleate | 40,5 |
| Creaderm | Lipodermanol OL | Decyl Olivate | 40,3 |
| *Henkel* | *Cetiol S* | Dioctylcyclohexane | 39,0 |
| DEA Mineralöl (Lief. Hansen & Rosenthal) Tudapetrol | Pionier 2071 | Mineral Oil | 38.3 |
| WITCO BV | Hydrobrite 1000 PO | Paraffinum Liquidum | 37,6 |
| Goldschmidt | Tegosoft HP | Isocetyl Palmitate | 36,2 |
| Condea Chemie | Isofol Ester 1693 | | 33,5 |
| Condea Chemie | Isofol Ester 1260 | | 33,0 |
| Dow Coming | Dow Coming Fluid 245 | Cyclopentasiloxan | 32,3 |
| Unichema | Prisorine 2036 | Octyl Isostearate | 31.6 |
| Henkel Cognis | Cetiol CC | Dicaprylyl Carbonate | 31,7 |
| ALZO (ROVI) | Dermol 99 | Trimethylhexyl Isononanoate | 31,1 |
| ALZO (ROVI) | Dermol 89 | 2- Ethylhexyl Isononanoate | 31,0 |
| Henkel Cognis | Cetiol OE | Dicaprylyl Ether | 30,9 |
| | *Dihexylcarbonat* | Dihexyl Carbonate | 30,9 |
| Albemarle S.A. | Silkflo 366 NF | Polydecene | 30,1 |
| Unichema | Estol 1540 EHC | Octyl Cocoate | 30.0 |

Erfindungsgemäß besonders bevorzugte unpolare Ölkomponenten sind insbesondere: Mineralöl, Squalan, Polydecen, Vaseline, Paraffin, Isohexadecan, Cyclomethicon, Dimeticon, und Phenyltrimethicon.

Neben unpolaren Ölkomponenten kann die erfindungsgemäße Emulsion auch polare und mittel-polare Öle/Lipide enthalten. Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Besonders vorteilhafte polare Ölkomponenten im Sinne der vorliegenden Erfindung sind alle nativen Ölkomponenten, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Hersteller** | **Handelsname** | **INCI-Name** | **Polarität [mN/m]** |
|---|---|---|---|
| Condea Chemie | Isofol 14 T | Butyl Decanol (+) Hexyl Octanol (+) Hexyl Decanol (+) Butyl Octanol | 19,8 |
| Lipochemicals INC. /USA (Induchem) | Lipovol MOS-130 | Tridecyl Stearate(+) Tridecyl Trimellitate(+) Dipentaerythrityl Hexacaprylate/Hexacaprate | 19,4 |
| | Ricinusoel | | 19,2 |
| CONDEA Chemie | Isofol Ester 0604 | | 19,1 |
| Huels CONDEA Chemie | Miglyol 840 | Propylene Glycol Dicaprylate/Dicaprate | 18,7 |
| *CONDEA Chemie* | Isofol 12 | Butyl Octanol | 17,4 |
| Goldschmidt | Tegosoft SH | Stearyl Heptanoate | 17,8 |
| | Avocadooel | | 14,5 |
| Henkel Cognis | Cetiol B | Dibutyl Adipate | 14,3 |
| ALZO (ROVI) | Dermol488 | PEG 2 Diethylenhexanoate | 10,1 |
| Condea Augusta S.P.A. | Cosmacol ELI | C12-13 Alkyl Lactate | 8,8 |
| ALZO (ROVI) | Dermol 489 | Diethylen Glycol Dioctanoate(/ Diisononanoate | 8,6 |
| Condea Augusta S.P.A. | Cosmacol ETI | Di-C12/13 Alkyl Tartrate | 7,1 |
| Henkel Cognis | Emerest 2384 | Propylene Glycol Monoisostearate | 6,2 |
| Henkel Cognis | Myritol 331 | Cocoglycerides | 5,1 |
| Unichema | Prisorine 2041 GTIS | Triisostearin | 2,4 |

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 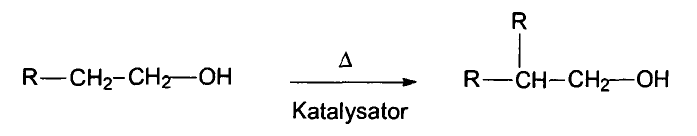 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 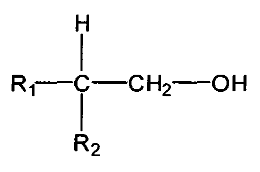 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 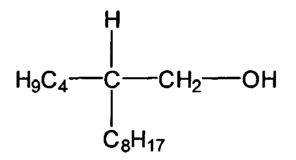 und ist beispielsweise unter der Handelsbezeichnung Isofol^{®} 12 von der Gesellschaft Condea Chemie GmbH erhältlich - und das 2-Hexyldecanol - es hat die chemische Struktur 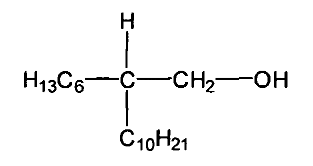 und ist beispielsweise unter der Handelsbezeichnung Isofol^{®} 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol^{®} 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 - 15,0 Gew.-% gewählt, bezogen auf das Gesamtgewicht der Zubereitungen.

Auch Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Ölkomponenten im Sinne der vorliegenden Erfindung sind die im folgenden aufgelisteten Substanzen:

| **Hersteller** | **Handelsname** | **INCI-Name** | **Polarit ät [mN/m]** |
|---|---|---|---|
| Stearinerie Dubois Fils | DUB VCl 10 | Isodecyl Neopentanoate | 29,9 |
| ALZO (ROVI) | Dermol IHD | Isohexyldecanoate | 29,7 |
| ALZO (ROVI) | Dermol 108 | Isodecyl Octanoate | 29,6 |
| | Dihexyl Ether | Dihexyl Ether | 29,2 |
| ALZO (ROVI) | Dermol 109 | Isodecyl 3,5,5 Trimethyl Hexanoate | 29,1 |
| Henkel Cognis | Cetiol SN | Cetearyl Isononanoate | 28,6 |
| Unichema | Isopropylpalmitat | Isopropylpalmitat | 28,8 |
| Dow Corning | DC Fluid 345 | Cyclomethicone | 28,5 |
| Dow Corning | Dow Corning Fluid 244 | Cyclopolydimethylsiloxan | 28,5 |
| Nikko Chemicals Superior Jojoba Oil Gold | Jojobaöl Gold | | 26,2 |
| Wacker | Wacker AK 100 | Dimethicone | 26,9 |
| ALZO (ROVI) | Dermol 98 | 2- Ethylhexanosäure 3,5,5 Trimethylester | 26,2 |
| Dow Corning | Dow Coming Fluid 246 | Offen | 25,3 |
| Henkel Cognis | Eutanol G | Octyldodecanol | 24,8 |
| Condea Chemie | Isofol 16 | Hexyl Decanol | 24,3 |
| ALZO (ROVI) | Dermol 139 | Isotridecyl 3,5,5 Trimethylhexanonanoate | 24,5 |
| Henkel Cognis | *Cetiol PGL* | Hexyldecanol (+) Hexyl Decyl Laurate | 24,3 |
| | Cegesoft C24 | Octyl Palmitate | 23,1 |
| Gattefossé | M.O.D. | Octyldodeceyl Myristate | 22,1 |
| | Macadamia Nut Oil | | 22,1 |
| Bayer AG, Dow Coming | *Silikonöl VP 1120* | Phenyl Trimethicone | 22,7 |
| CONDEA Chemie | Isocarb 12 | Butyl Octanoicacid | 22,1 |
| Henkel Cognis | Isopropylstearat | Isopropyl Stearate | 21,9 |
| WITCO, Goldschmidt | Finsolv TN | C12-15 Alkyl Benzoate | 21,8 |
| Dr. Straetmans | Dermofeel BGC | Butylene Glycol Caprylate/Caprate | 21,5 |
| Unichema Huels | Miglyol 812 | Caprylic/Capric Triglyceride | 21,3 |
| Trivent (über S. Black) | Trivent OCG | Tricaprylin | 20,2 |
| ALZO (ROVI) | Dermol 866 | PEG" Diethylhexanoate/ Diisononanoate/ Ethylhexyl Isononanoate | 20,1 |

Es ist insbesondere vorteilhaft, Gemische aus höher- und niederpolaren Ölkomponenten und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat), und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen. Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

### Silikone

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

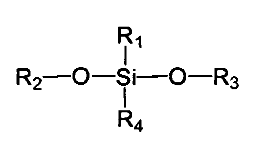

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 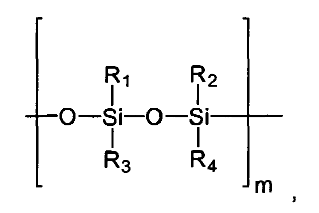 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 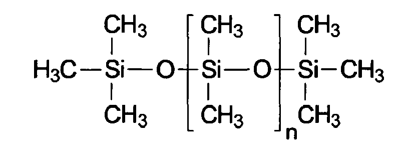 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

| **Hersteller** | **Handelsname** | **INCI-Name** | **Polarität [mN/m ]** |
|---|---|---|---|
| Wacker | Wacker Silikonöl AK 100 | Polydimethylsiloxan | 26,9 |
| Wacker | Wacker Silikonöl AK 50 | Polydimethylsiloxan | 46,5 |
| Wacker | Wacker Silikonöl AK 35 | Polydimethylsiloxan | 42,4 |
| Wacker | Wacker Silikonöl AK 20 | Polydimethylsiloxan | 40,9 |
| Dow Corning | Dow Corning Fluid 245 | Cyclopentasiloxan | 32,3 |
| Dow Corning | Dow Corning Fluid 345 | Cyclomethicone | 28,5 |

Erfindungsgemäß vorteilhaft einzusetzende cyclische Silicone werden im allgemeinen durch Strukturelemente charakterisiert, wie folgt 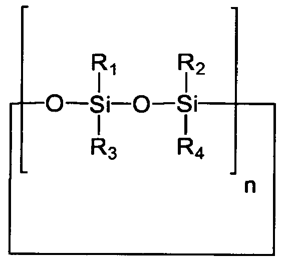 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Ölkomponentenn zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Die Wasserphase der erfindungsgemäßen Emulsion kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie Moisturizer.

Erfindungsgemäß besonders bevorzugt ist es, wenn die erfindungsgemäße Emulsion als Schaumstabilisator ein Polymer aus der Grupppe der Celluloseether, insbesondere Ethylcellulose oder Hydroxyethylmethylcellulose, enthält.

Die kosmetisch oder dermatologisch einsetzbare Emulsion gemäß der Erfindung kann kosmetische Hilfsstoffe enthalten, wie sie üblicherweise in solchen Zubereitungen verwendet werden, z. B. Konservierungsmittel, Konservierungshelfer, Bakterizide, Parfüme, Farbstoffe, Pigmente, die eine färbende Wirkung haben, anfeuchtende und/oder feuchthaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Als Feuchthaltemittel (Moisturizer) werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Erfindungsgemäß bevorzugte Ausführungsformen der vorliegenden Erfindung sind dadurch gekennzeichnet, dass sie einen oder mehrere Feuchthaltemittel gewählt aus der Gruppe Glycerin, Milchsäure, Pyrrolidoncarbonsäure und Harnstoff in einer Gesamtkonzentration von bis Gewichts- 0,1-15%, bezogen auf das Gesamtgewicht der Emulsion, enthält.

Vorteilhafte Konservierungsmittel im Sinne der vorliegenden Erfindung sind beispielsweise Formaldehydabspalter (wie z. B. DMDM Hydantoin), lodopropylbutylcarbamate (z. B. die unter den Handelsbezeichnungen Koncyl-L, Koncyl-S und Konkaben LMB von der Fa. Lonza erhältlichen), Parabene, Phenoxyethanol, Ethanol, Benzoesäure und dergleichen mehr.

Besonders vorteilhafte Emulsionen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, y -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Eine erstaunliche Eigenschaft der erfindungsgemäßen Emulsion ist, dass diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Es war insbesondere erstaunlich, dass die erfindungsgemäßen kosmetischen oder dermatologischen Zubereitungen wasserempfindliche, hydrophile Wirkstoffe - insbesondere Ascorbinsäure - besser gegen den Zerfall stabilisieren als die Zubereitungen des Standes der Technik und dass sie ferner ausgezeichnete kosmetische und dermatologische Transportmittel für Ascorbinsäure und/oder Ascorbylverbindungen darstellen.

Die Menge der Antioxidantien (eine oder mehrere Verbindungen) in den Zubereitungen beträgt vorzugsweise 0,0001 bis 30 Gew.-%, besonders bevorzugt 0,0005 bis 10 Gew.-%, insbesondere 0,001 bis 5 Gew.-%, bezogen auf das Gesamtgewicht der Zubereitung.

Ferner bevorzugte Antioxidantien sind Vitamin E und dessen Derivate sowie Vitamin A und dessen Derivate. Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine der D-Reihe, Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit^{®} und Neocerit^{®}.

Vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

### Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

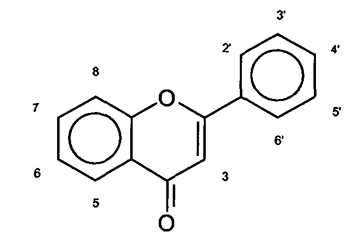

Einige der wichtigeren Flavone, welche auch bevorzugt in erfindungsgemäßen Zubereitungen eingesetz werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel 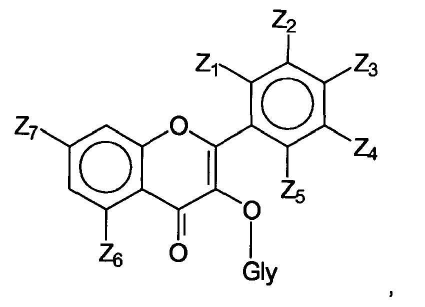 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 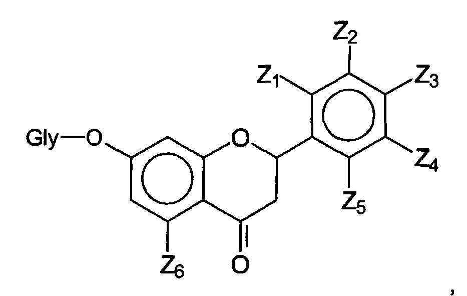 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 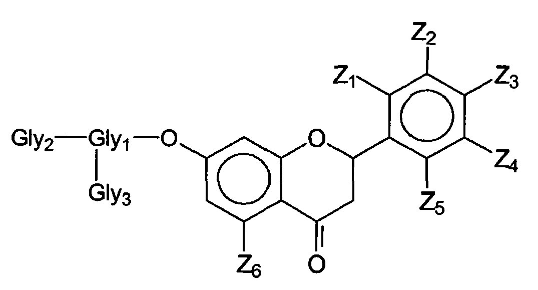 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

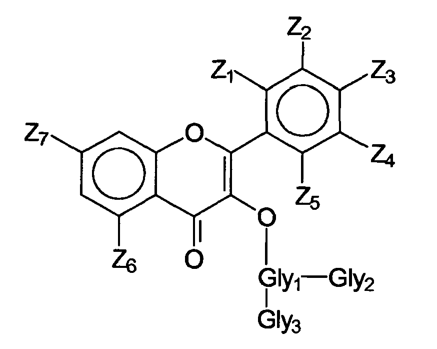

Besonders vorteilhaft werden die erfindungsgemäßen Flavonglycoside aus der Gruppe, welche durch die folgende Struktur wiedergegeben werden: 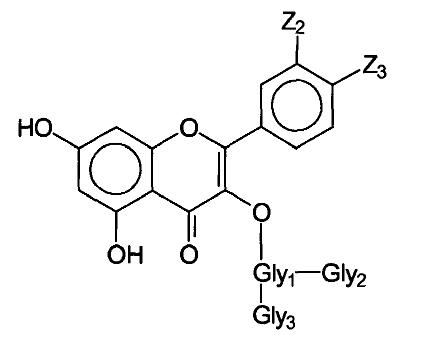 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel 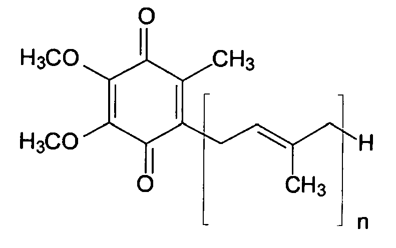 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10. Besonders vorteilhaft ist Coenzym Q10.

Plastochinone weisen die allgemeine Strukturformel 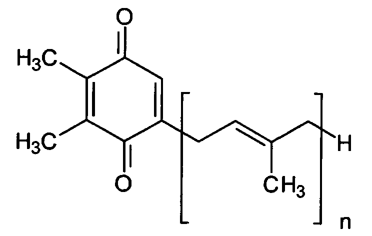 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung.

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-butter-säurebetain]. Auch Acyl-Carnitine, welche gewählt aus der Gruppe der Substanzen der folgenden allgemeinen Strukturformel 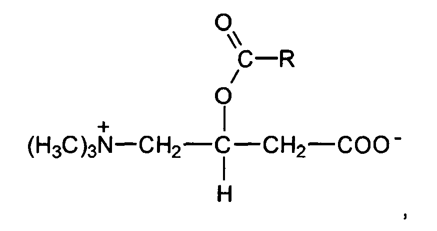 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Enantiomere (D-und L-Form) sind vorteilhaft im Sinne der vorliegenden Erfindung zu verwenden. Es kann auch von Vorteil sein, beliebige Enantiomerengemische, beispielsweise ein Racemat aus D-und L-Form, zu verwenden.

Weitere vorteilhafte Wirkstoffe sind Sericosid, Pyridoxol, Vitamin K, Biotin, Coffein und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Erfindungsgemäß besonders bevorzugte Ausführungsformen der vorliegenden Erfindung sind dadurch gekennzeichnet, dass sie einen oder mehrere Wirkstoffe, gewählt aus der Gruppe Flavonoide, Isoflavonoide, Kreatin und Derivate, Panthenol, Licochalcone, Ubichinon Q10, Enzyme aus der Gruppe der Lipasen und Esterasen, Carnitin und Derivate, Niacinamid oder deren Derivate in einer Gesamtkonzentration von 0,001 bis 5 Gewichts-%, bezogen auf das Gesamtgewicht der Emulsion, enthalten.

Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von (Trockenheits-) Fältchen,
b) Juckreiz und
c) verminderte Rückfettung durch Talgdrüsen (z. B. nach dem Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Erstaunlicherweise können ausgewählte erfindungsgemäße Rezepturen auch eine Antifaltenwirkung aufweisen bzw. die Wirkung bekannter Antifaltenwirkstoffe erheblich steigern. Dementsprechend eignen sich Formulierungen im Sinne der vorliegenden Erfindung insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

In einer besonderen Ausführungsform betrifft die vorliegende Erfindung daher Produkte zur Pflege der auf natürliche Weise gealterten Haut, sowie zur Behandlung der Folgeschäden der Lichtalterung, insbesondere der unter a) bis g) aufgeführten Phänomene.

Die erfindungsgemäßen Emulsionen können Farbstoffe und/oder Farbpigmente enthalten, insbesondere wenn sie in Form von dekorativen Kosmetika vorliegen. Die Farbstoffe und - pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem *Rowe Colour Index,* 3. *Auflage, Society of Dyers and Colourists, Bradford, England,* 1971 zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| | | |
|---|---|---|
| | | |
| | TiO₂: 40 - 60 nm | silber |
| | TiO₂: 60 - 80 nm | gelb |
| | TiO₂: 80 -100 nm | rot |
| | TiO₂: 100 -140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise neben einer oder mehreren erfindungsgemäßen UV-Filtersubstanzen zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein,
wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z-Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |
| Eusolex T-aqua | Wasser/Alumina/Natriummetaphosphat | Merck |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex^{®} 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 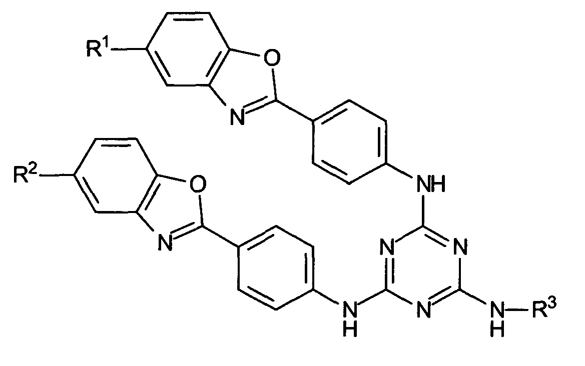 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 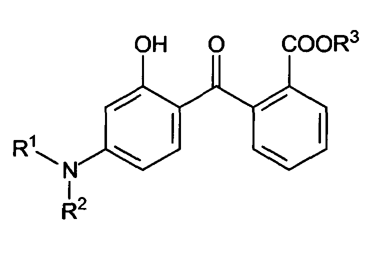 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.
   Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher unter dem Handelsnamen Uvinul A Plus bei der Fa. BASF erhältlich ist.
   Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.
   Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Vorteilhafte wasserlösliche Filtersubstanzen sind z. B.: Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander. Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 0,1 Gew.-% bis zu 5 Gew.-%, insbesondere 0,5 bis 3 Gew.-%, jeweils bezogen auf das Gesamtgewicht der Zubereitung.

Die erfindungsgemäßen Emulsionen enthalten erfindungsgemäß vorteilhaft ein oder mehrere Tenside. Ein Gehalt an Tensiden ist insbesondere dann erfindungsgemäß von Vorteil, wenn die Emulsion mit Hilfe eines Treibgases aufgeschäumt werden soll.

Erfindungsgemäß bevorzugte Emulsionen sind dadurch gekennzeichnet, dass sie ein oder mehrere Tenside gewählt aus der Gruppe N-Acylsarcosinate mit einer Kettenlänge von 10 bis 28 Kohlenstoffatomen, Polyglycoside mit einer Kettenlänge von 10 bis 28 Kohlenstoffatomen und Acylglutamate mit einer Kettenlänge von 10 bis 28 Kohlenstoffatomen, in einer Gesamtkonzentration von 0,5 bis 2 Gewichts-%, bezogen auf das Gesamtgewicht der Emulsion, enthalten.

Erfindungsgemäß sind auch erfindungsgemäße Emulsionen im aufgeschäumten Zustand. Dieser erfindungsgemäße kosmetische Schaum aus einer Emulsion und einem Treibgas bzw. einer Treibgasmischung ist insbesondere dann erfindungsgemäß bevorzugt, wenn das Treibgas/die Treibgasmischung gewählt wird aus der Gruppe der Treibgase/Treibgasmischungen Luft, Kohlendioxid, Sauerstoff, Stickstoff, Dimethylether, Propan, Butan, Isobutan und Mischungen von Propan, Butan und/oder Isobutan.

Erfindungsgemäß ist ferner ein Schaumspender enthaltend eine erfindungsgemäße Emulsion.

Die schäumbaren Emulsionen gemäß der Erfindung können beispielsweise aus Aerosolbehältern entnommen und dabei aufgeschäumt werden. Erfindungsgemäße Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Derartige Behälter können mit Ventilen sehr unterschiedlicher Bauart ausgestattet sein, die die Entnahme des Inhalts als Schaum ermöglichen.

Als Druckgasbehälter kommen im Sinne der vorliegenden Erfindung vor allem zylindrische Gefäße aus Metall (Aluminium, Weißblech, Inhalt <1000 mL), geschütztem bzw. nichtsplitterndem Glas oder Kunststoff (Inhalt < 220 mL) bzw. splitterndem Glas oder Kunststoff (Inhalt < 150 mL) in Frage, bei deren Auswahl Druck- und Bruchfestigkeit, Korrosionsbeständigkeit, leichte Füllbarkeit, ggf. Sterilisierbarkeit usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50 °C ist 12 bar und das maximale Füllvolumen bei dieser Temperatur ca. 90 % des Gesamtvolumens. Für Glas- und Kunststoffdosen gelten niedrigere, von der Behältergröße und dem Treibmittel (ob verflüssigtes, verdichtetes oder gelöstes Gas) abhängige Werte für den Betriebsdruck.

Besonders vorteilhaft im Sinne der vorliegenden Erfindung sind Dosen aus Weißblech, Aluminium und Glas. Aus Korrosionsschutzgründen können Metalldosen innen lackiert sein (silber- oder goldlackiert), wozu alle handelsüblichen Innenschutzlacke geeignet sind. Bevorzugt im Sinne der vorliegenden Erfindung sind Polyester-, Epoxyphenol- so wie Polyamidimidlacke. Auch Folienkaschierungen aus Polyethylen (PE), Polypropylen (PP) und/oder Polyethylenterephthalat (PET) im Innern der Dosen sind vorteilhaft, insbesondere für Dosen aus Weißblech.

Die Druckgasbehälter sind üblicherweise ein- oder zwei-, meist aber dreiteilig zylindrisch, konisch oder anders geformt. Werden Kunststoffe als Sprüh-Behältermaterial verwendet, so sollten diese Chemikalien- und Sterilisationstemperatur-beständig, gasdicht, schlagfest und gegen Innendrücke über 12 bar stabil sein. Prinzipiell für Sprüh-Behälter-Zwecke geeignet sind Polyacetale und Polyamide.

Der innere Aufbau der Sprüh-Dosen sowie die Ventilkonstruktion sind je nach VerwendungsZweck und der physikalischen Beschaffenheit des Inhalts - z. B. ob als Zwei- oder als Dreiphasensystem - sehr variantenreich und können vom Fachmann durch einfaches Ausprobieren ohnen erfinderisches Zutun ermittelt werden. Für geeignete Ausführungsformen sei auf das "Aerosol Technologie Handbuch der Aersosol-Verpackung" hingewiesen *(Wolfgang Tauscher, Melcher Verlag GmbH Heidelberg*/*München, 1996*).

Erfindungsgemäß vorteilhafte Ventile können mit oder ohne Steigrohr ausgebildet sein. Die Einzelteile, aus welchen erfindungsgemäße Ventile üblicherweise aufgebaut sind, bestehen vorzugsweise aus den folgenden Materialien:

| | |
|---|---|
| Teller: | Weißblech: blank, gold- bzw. klarlackiert, folienkaschiert (PE, PP oder PET) Aluminium: blank, silber- oder goldlackiert, verschiedene Lackvarianten, Stoner-Mudge-Ausführung |
| Dichtung: | natürliche bzw. synthetische Elastomere bzw. thermoplastische (Sleeve-Gaskets, folienkaschiert aus PE oder PP) Innen- und Aussendichtungen, z. B. aus Perbunan, Buna, Neopren, Butyl, CLB, LDPE, Viton, EPDM, Chlorbutyl, Brombutyl und/oder diversen Compounds |
| Kegel: | PA, POM, Messing sowie diversen Sondermaterialen, Standardbohrungen (z. B.: 0,25 bis 0,70 mm oder 2 x 0,45 bis 2 x 1,00 mm), verschiedene Schaftdurchmesser |
| Feder: | Metall, besonders bevorzugt V2A, rostfreier Stahl; Kunststoff und auch Elastomer |
| Gehäuse: | Standard und Impact VPH-Bohrungen, RPT-Bohrungen oder geschlitzt für Überkopf-Anwendungen Materialien: z. B. Polyacetal, PA, PE, POM und dergleichen mehr |
| Steigrohr: | Kunststoff (Polymer Resin), z.B. PE, PP, PA oder Polycarbonat |

Vorteilhafte Sprühköpfe im Sinne der vorliegenden Erfindung sind beispielsweise Schaumköpfe für die aufrechte Anwendung (Dose senkrecht halten) oder Schaumköpfe für die Überkopf-Anwendung mit einem oder mehreren Kanälen.

Als Treibmittel sind die üblichen klassischen" leichtflüchtigen, verflüssigten Treibgase, wie beispielsweise Dimethylether (DME) und/oder lineare oder verzweigtkettige Kohlenwasserstoffe mit zwei bis fünf Kohlenstoffatomen (wie insbesondere Ethan, Propan, Butan, Isobutan und/oder Pentan) geeignet, die allein oder in Mischung miteinander eingesetzt werden können.

Auch Druckluft sowie weitere unter Druck befindliche Gase wie Luft, Sauerstoff, Stickstoff, Wasserstoff, Helium, Krypton, Xenon, Radon, Argon, Lachgas (N₂O) und Kohlendioxid (CO₂) sind vorteilhaft im Sinne der vorliegenden Erfindung als Treibgase (sowohl einzeln als in beliebigen Mischungen miteinander) zu verwenden.

Natürlich weiß der Fachmann, dass es weitere an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere halogenierte (mit Fluor, Chlor, Brom, Iod und/oder Astat substituierte) Kohlenwasserstoffe wie beispielsweise Fluorkohlenwasserstoffe und Fluorchlorkohlenwasserstoffe (FCKW).

Die genannten Gase können im Sinne der vorliegenden Erfindung jeweils einzeln oder in beliebigen Mischungen zueinander verwendet werden.

Vorteilhaft im Sinne der vorliegenden Erfindung wird der Volumenanteil an Treibgas aus dem Bereich von 0,1 bis 30 Vol.-%, bezogen auf das Gesamtvolumen aus Füllgut und Treibgas gewählt (entsprechend einem Volumenanteil von 70 bis 99,9 Vol.-% Füllgut).

Besonders vorteilhafte, feincremige und reichhaltige Schäume sind erhältlich, wenn die erfindungsgemäßen Zubereitungen mit Hilfe von linearen oder verzweigtkettigen, halogenierten oder nicht-halogenierten Kohlenwasserstoffen aufgeschäumt werden. Ganz besonders vorteilhafte Schäume sind durch Aufschäumen der erfindungsgemäßen Zubereitungen mit Kohlendioxid, Sauerstoff, Druckluft und/oder Stickstoff erhältlich.

Erfindungsgemäß ist auch das Verfahren zur Herstellung von kosmetischen Schäumen, welches dadurch gekennzeichnet ist, dass eine erfindungsgemäße Emulsion mit einem Treibgas bzw. einer Treibgasmischung gewählt aus der Gruppe Luft, Kohlendioxid, Sauerstoff, Stickstoff, Dimethylether, Propan, Butan, Isobutan und Mischungen von Propan, Butan und/oder Isobutan aufgeschäumt wird.

Erfindungsgemäß ist die kosmetische, nicht therapeutische Verwendung einer erfindungsgemäßen Emulsion zur Reinigung und Pflege der Haut, insbesondere der Gesichtshaut.

Die erfindungsgemäßen kosmetischen und/oder dermatologischen Emulsionen und daraus erhältliche Schäume können können dem kosmetischen und/oder dermatologischen Lichtschutz, ferner zur Behandlung der Haut und/oder der Haare und als Schminkprodukt in der dekorativen Kosmetik dienen. Eine weitere vorteilhafte Ausführungsform der vorliegenden Erfindung besteht in After-Sun-Produkten.

Ebenso wie Emulsionen von flüssiger und fester Konsistenz als kosmetische Reinigungslotionen bzw. Reinigungscremes Verwendung finden, können auch die aus erfindungsgemäßen Zubereitungen erhältlichen Schäume "Reinigungsschäume" darstellen, welche beispielsweise zum Entfernen von Schminken und/oder Make-up oder als milder Waschschaum - ggf. auch für unreine Haut - verwendet werden können. Derartige Reinigungsschäume können vorteilhaft ferner als sogenannte "rinse off" Präparate angewendet werden, welche nach der Anwendung von der Haut abgespült werden

Aus erfindungsgemäßen kosmetischen und/oder dermatologischen Zubereitungen erhältliche Schäume können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Zur Anwendung werden die erfindungsgemäßen kosmetischen und dermatologischen Zubereitungen und/oder daraus erhältliche Schäume in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

### (1) O/W Emulsionschaum

| | Gew.-% |
|---|---|
| Glycerylstearat (selbstemulgierend) | 1,00 |
| Cetearylalkohol | 2,00 |
| PEG-40 Ricinusöl | 0,30 |
| Natriumcetearylsulfat | 0,15 |
| Mineralöl | 6,00 |
| Cocosglyceride | 1,00 |
| Isohexadecan | 1,00 |
| Vaseline | 1,00 |
| Dimethicon (linear) | 2,00 |
| C12-15 Alkylbenzoat | 1,00 |
| Carageenan | 0,20 |
| Xanthan Gum | 0,10 |
| Kreatin | 0,20 |
| Panthenol | 0,50 |
| Tocopherylacetat | 0,20 |
| Glycerin | 5,00 |
| Methylpropandiol | 2,00 |
| Allantoin | 0,50 |
| Phenoxyethanol | 0,50 |
| Methylparaben | 0,30 |
| Propylparaben | 0,20 |
| Füllstoffe/ Additive (Iminodisuccinat, Aloe | 0,50 |
| Vera, Distärkephosphat, Chitosan) | |
| Treibgas | q.s. |
| Parfum | q.s. |
| Wasser | 100,00 |
| **PH = 6.5** | |

### (2) O/W Emulsionschaum

| | **Gew.-%** |
|---|---|
| Glycerylstearat (selbstemulgierend) | 0,50 |
| Cetearylalkohol | 2,50 |
| PEG-40 Ricinusöl | 0,50 |
| Natriumcetearylsulfat | 0,30 |
| Mineralöl | 5,00 |
| Cocosglyceride | 1,00 |
| Isohexadecan | 1,00 |
| Ethylhexylstearat | 1,00 |
| Dimethicon (linear) | 3,00 |
| C12-15 Alkylbenzoat | 2,00 |
| Carageenan | 0,20 |
| Xanthan Gum | 0,10 |
| Kreatin | 0,20 |
| Hydroxypropylmethylcellulose | 0,50 |
| Panthenol | 0,50 |
| Ubichinon (Q10) | 0,05 |
| Glycerin | 5,00 |
| Methylpropandiol | 2,00 |
| Bisabolol | 0,10 |
| Phenoxyethanol | 0,40 |
| Parabene | 0,40 |
| lodopropylbutylcarbamat | 0,05 |
| Füllstoffe/ Additive (EDTA, Aloe Vera, | 0,50 |
| Distärkephosphat, Chitosan) | |
| Treibgas | q.s. |
| Parfum | q.s. |
| Wasser | 100,00 |
| **PH = 6.0** | |

### (3) O/W Emulsionschaum

| | **Gew.-%** |
|---|---|
| Glycerylstearat (selbstemulgierend) | 0,50 |
| Cetearylalkohol | 2,50 |
| PEG-40 Ricinusöl | 0,40 |
| Natriumcetearylsulfat | 0,10 |
| Mineralöl | 5,00 |
| Isohexadecan | 1,00 |
| Ethylhexylstearat | 1,00 |
| Vaseline | 2,00 |
| C12-15 Alkylbenzoat | 2,00 |
| Carageenan | 0,20 |
| Xanthan Gum | 0,10 |
| Octyl methoxycinnamat | 2,00 |
| Hydroxypropylmethylcellulose | 0,50 |
| Panthenol | 0,50 |
| Ubichinon (Q10) | 0,05 |
| Glycerin | 5,00 |
| Ethylhexylglycerin | 1,00 |
| Bisabolol | 0,10 |
| Phenoxyethanol | 0,50 |
| Parabene | 0,40 |
| lodopropylbutylcarbamat | 0,05 |
| Füllstoffe/ Additive (EDTA, Aloe Vera, | 0,50 |
| Distärkephosphat, Chitosan) | |
| Treibgas | q.s. |
| Parfum | q.s. |
| Wasser | 100,00 |
| **PH = 6.0** | |

### Beispiele

### (4) O/W Emulsionschaum

| | **Gew.-%** |
|---|---|
| Glycerylstearat (selbstemulgierend) | 1,00 |
| Cetearylalkohol + Cetearylsulfat + PEG-40 | 3,00 |
| Ricinusöl (Emulgade F) | |
| Jojobaöl | 0,50 |
| Mineralöl | 5,00 |
| Cocosglyceride | 1,00 |
| Isohexadecan | 2,00 |
| Vaseline | 1,00 |
| Dimethicon (linear) | 2,00 |
| C12-15 Alkylbenzoat | 1,00 |
| Hydroxypropylmethylcellulose | 0,50 |
| Carageenan | 0,20 |
| Xanthan Gum | 0,10 |
| Kreatin | 0,20 |
| Panthenol | 0,50 |
| Tocopherylacetat | 0,20 |
| Glycerin | 5,00 |
| Methylpropandiol | 2,00 |
| Bisabolol | 0,10 |
| Phenoxyethanol | 0,50 |
| Methylparaben | 0,30 |
| Propylparaben | 0,20 |
| Additive (EDTA, Distärkephosphat) | 0,50 |
| Treibgas | q.s. |
| Parfum | q.s. |
| Wasser | 100,00 |
| **PH = 6.5** | |

## Patentansprüche

1. Kosmetische O/W-Emulsion enthaltend eine Emulgatorkombination aus
a) Glycerylmonostearat in einer Konzentration von 0,1 bis 3,0 Gewichts-%,
b) Fettalkohol in einer Konzentration von 0,1 bis 4,0 Gewichts-%,
c) PEG-40 Rizinusöl in einer Konzentration von 0,1 bis 3,0 Gewichts-%,
d) Natriumcetylstearylsulfat in einer Konzentration von 0,01 bis 1,0 Gewichts-%, jeweils bezogen auf das Gesamtgewicht der Emulsion, wobei die Ölphase der Emulsion **dadurch gekennzeichnet ist, dass** sie 1 bis 90 Gewichts-%, bezogen auf das Gesamtgewicht der Ölphase, unpolare Ölkomponenten enthält.

2. Kosmetische O/W-Emulsion nach Anspruch 1, **dadurch gekennzeichnet, dass** die Gesamtmenge an Emulgatoren a), b) c) und d) von 0,5 bis 8,0% Gewichts-%, bezogen auf das Gesamtgewicht der Emulsion, beträgt.

3. Emulsion nach einem der vorhergehenden Ansprüche, **dadurch gekennzeichnet, dass** die unpolaren Ölkomponenten gewählt werden aus der Gruppe Mineralöl, Squalan, Polydecen, Vaseline, Paraffinen, Isohexadecan, Cyclomethicon, Dimeticon, Phenyltrimethicon.

4. Emulsion nach einem der vorhergehenden Ansprüche, **dadurch gekennzeichnet, dass** sie einen oder mehrere Wirkstoffe, gewählt aus der Gruppe Flavonoide, Isoflavonoide, Kreatin und Derivate, Panthenol, Licochalcone, Ubichinon Q10, Enzyme aus der Gruppe der Lipasen und Esterasen, Carnitin und Derivate, Niacinamid in einer Gesamtkonzentration von 0,001 bis 5 Gewichts-%, bezogen auf das Gesamtgewicht der Emulsion, enthält.

5. Emulsion nach einem der vorhergehenden Ansprüche, **dadurch gekennzeichnet, dass** sie einen oder mehrere Feuchthaltemittel gewählt aus der Gruppe Glycerin, Milchsäure, Pyrrolidoncarbonsäure und Harnstoff in einer Gesamtkonzentration von 0,1 bis 15 Gewichts-%, bezogen auf das Gesamtgewicht der Emulsion, enthält.

6. Emulsion nach einem der vorhergehenden Ansprüche, **dadurch gekennzeichnet, dass** als Fettalkohol b) Cetostearylalkohol eingesetzt wird.

7. Kosmetischer Schaum aus einer Emulsion nach einem der Ansprüche 1 bis 6 und einem Treibgas bzw. einer Treibgasmischung gewählt aus der Gruppe der Treibgase/Treibgasmischungen Luft, Kohlendioxid, Sauerstoff, Stickstoff, Dimethylether, Propan, Butan, Isobutan und Mischungen von Propan, Butan und/oder Isobutan.

8. Schaumspender enthaltend eine Emulsion nach einem der Ansprüche 1 bis 6.

9. Verfahren zur Herstellung von kosmetischen Schäumen, **dadurch gekennzeichnet, dass** eine Emulsion nach einem der Ansprüche 1 bis 6 mit einem Treibgas bzw. einer Treibgasmischung gewählt aus der Gruppe Luft, Kohlendioxid, Sauerstoff, Stickstoff, Dimethylether, Propan, Butan, Isobutan und Mischungen von Propan, Butan und/oder Isobutan aufgeschäumt wird.

10. Kosmetische, nicht-therapeutische verwendung einer Emulsion nach einem der vorhergehenden Ansprüche zur Pflege und Reinigung der Haut, insbesondere der Gesichtshaut.

## Claims

1. Cosmetic O/W emulsion comprising an emulsifier combination of
a) glyceryl monostearate in a concentration from 0.1 to 3.0% by weight,
b) fatty alcohol in a concentration of from 0.1 to 4.0% by weight,
c) PEG-40 castor oil in a concentration of from 0.1 to 3.0% by weight,
d) sodium cetylstearyl sulphate in a concentration of from 0.01 to 1.0% by weight,
in each case based on the total weight of the emulsion, where the oil phase of the emulsion is **characterized in that** it comprises 1 to 90% by weight, based on the total weight of the oil phase, of nonpolar oil components.

2. Cosmetic O/W emulsion according to Claim 1, **characterized in that** the total amount of emulsifiers a), b), c) and d) is from 0.5 to 8.0% by weight, based on the total weight of the emulsion.

3. Emulsion according to one of the preceding claims, **characterized in that** the nonpolar oil components are chosen from the group consisting of mineral oil, squalane, polydecene, Vaseline, paraffins, isohexadecane, cyclomethicone, dimethicone, phenyltrimethicone.

4. Emulsion according to one of the preceding claims, **characterized in that** it comprises one or more active ingredients chosen from the group consisting of flavonoids, isoflavonoids, creatine and derivatives, panthenol, licochalcones, ubiquinone Q10, enzymes from the group of lipases and esterases, carnitine and derivatives, niacinamide in a total concentration of from 0.001 to 5% by weight, based on the total weight of the emulsion.

5. Emulsion according to one of the preceding claims, **characterized in that** it comprises one or more humectants chosen from the group consisting of glycerol, lactic acid, pyrrolidonecarboxylic acid and urea in a total concentration of from 0.1 to 15% by weight, based on the total weight of the emulsion.

6. Emulsion according to one of the preceding claims, **characterized in that** the fatty alcohol b) used is cetostearyl alcohol.

7. Cosmetic foam of an emulsion according to one of Claims 1 to 6 and a propellant gas or a propellant gas mixture chosen from the group of propellant gases/propellant gas mixtures consisting of air, carbon dioxide, oxygen, nitrogen, dimethyl ether, propane, butane, isobutane and mixtures of propane, butane and/or isobutane.

8. Foam dispenser comprising an emulsion according to one of Claims 1 to 6.

9. Method of producing cosmetic foams, **characterized in that** an emulsion according to one of Claims 1 to 6 is foamed using a propellant gas or a propellant gas mixture chosen from the group consisting of air, carbon dioxide, oxygen, nitrogen, dimethyl ether, propane, butane, isobutane and mixtures of propane, butane and/or isobutane.

10. Cosmetic, nontherapeutic use of an emulsion according to one of the preceding claims for the care and cleansing of the skin, in particular of the facial skin.

## Revendications

1. Emulsion H/E cosmétique contenant une association d'émulsifiants à base de
a) monostéarate de glycéryle à une concentration de 0,1 à 3,0 % en poids,
b) alcool gras à une concentration de 0,1 à 4,0 % en poids,
c) PEG-40-huile de ricin à une concentration de 0,1 à 3,0 % en poids,
d) cétylstéarylsulfate de sodium à une concentration de 0,01 à 1,0 % en poids,
chaque fois par rapport au poids total de l'émulsion, la phase huileuse de l'émulsion étant **caractérisée en ce qu'**elle contient de 1 à 90 % en poids, par rapport au poids total de la phase huileuse, de composants huileux non polaires.

2. Emulsion H/E cosmétique selon la revendication 1, **caractérisée en ce que** la quantité totale d'émulsifiants a), b), c) et d) va de 0,5 à 8,0 % en poids, par rapport au poids total de l'émulsion.

3. Emulsion selon l'une quelconque des revendications précédentes, **caractérisée en ce que** les composants huileux non polaires sont choisis dans le groupe constitué par l'huile minérale, le squalane, le polydécène, la vaseline, les paraffines, l'isohexadécane, la cyclométhicone, la diméthicone, la phényltriméthicone.

4. Emulsion selon l'une quelconque des revendications précédentes, **caractérisée en ce qu'**elle contient un ou plusieurs actifs choisis dans le groupe constitué par les flavonoïdes, les isoflavonoïdes, la créatine et ses dérivés, le panthénol, les licochalcones, l'ubiquinone Q10, des enzymes choisies dans le groupe des lipases et estérases, la camitine et ses dérivés, le niacinamide, à une concentration totale de 0,001 à 5 % en poids, par rapport au poids total de l'émulsion.

5. Emulsion selon l'une quelconque des revendications précédentes, **caractérisée en ce qu'**elle contient un ou plusieurs agents retenant l'humidité, choisis dans le groupe constitué par le glycérol, l'acide lactique, l'acide pyrrolidonecarboxylique et l'urée, à une concentration totale de 0,1 à 15 % en poids, par rapport au poids total de l'émulsion.

6. Emulsion selon l'une quelconque des revendications précédentes, **caractérisée en ce qu'**on utilise comme alcool gras b) l'alcool cétostéarylique.

7. Mousse cosmétique à base d'une émulsion selon l'une quelconque des revendications 1 à 6 et d'un gaz propulseur ou d'un mélange de gaz propulseurs choisis dans le groupe des gaz propulseurs/mélanges de gaz propulseurs air, dioxyde de carbone, oxygène, azote, éther diméthylique, propane, butane, isobutane et mélanges de propane, butane et/ou isobutane.

8. Distributeur de mousse contenant une émulsion selon l'une quelconque des revendications 1 à 6.

9. Procédé pour la production de mousses cosmétiques, **caractérisé en ce qu'**on transforme en mousse une émulsion selon l'une quelconque des revendications 1 à 6, au moyen d'un gaz propulseur ou d'un mélange de gaz propulseurs choisis dans le groupe constitué par l'air, le dioxyde de carbone, l'oxygène, l'azote, l'éther diméthylique, le propane, le butane, l'isobutane et les mélanges de propane, butane et/ou isobutane.

10. Utilisation cosmétique, non thérapeutique, d'une émulsion selon l'une quelconque des revendications précédentes, pour le soin et le nettoyage de la peau, en particulier de la peau du visage.
